(19) Europäisches Patentamt / European Patent Office / Office européen des brevets

(11) **EP 1 653 218 B1**

(12) **EUROPEAN PATENT SPECIFICATION**

(45) Date of publication and mention
of the grant of the patent:
**20.04.2016 Bulletin 2016/16**

(51) Int Cl.:
*A61B 5/1455* *(2006.01)*  *G01N 21/49* *(2006.01)*
*G01N 21/47* *(2006.01)*

(21) Application number: **04770890.4**

(86) International application number:
**PCT/JP2004/010497**

(22) Date of filing: **23.07.2004**

(87) International publication number:
**WO 2005/010504 (03.02.2005 Gazette 2005/05)**

(54) **DEVICE AND METHOD FOR MEASURING SCATTERING ABSORBER**

VORRICHTUNG UND VERFAHREN ZUR MESSUNG VON STREULICHT-ABSORBERN

DISPOSITIF ET METHODE POUR MESURER UN ELEMENT ABSORBANT LA LUMIERE DIFFUSEE

(84) Designated Contracting States:
**DE FR GB IT**

(30) Priority: **29.07.2003 JP 2003282067**

(43) Date of publication of application:
**03.05.2006 Bulletin 2006/18**

(73) Proprietor: **HAMAMATSU PHOTONICS K.K.**
**Hamamatsu-shi, Shizuoka-ken 435-8558 (JP)**

(72) Inventors:
• **YAMANAKA, Takeshi,**
**c/o Hamamatsu Photonics K.K.**
**Hamamatsu-shi,**
**Shizuoka 435-8558 (JP)**
• **UEDA, Yukio,**
**c/o Hamamatsu Photonics K.K.**
**Hamamatsu-shi,**
**Shizuoka 435-8558 (JP)**
• **YAMASHITA, Yutaka,**
**c/o Hamamatsu Photonics K.K.**
**Hamamatsu-shi,**
**Shizuoka 435-8558 (JP)**

(74) Representative: **Boult Wade Tennant**
**Verulam Gardens**
**70 Gray's Inn Road**
**London WC1X 8BT (GB)**

(56) References cited:
| | |
|---|---|
| EP-A- 0 290 278 | EP-A- 0 628 804 |
| EP-A- 0 758 082 | EP-A- 1 245 192 |
| JP-A- 6 343 625 | JP-A- 9 184 800 |
| JP-A- 9 184 800 | JP-A- 2001 337 033 |
| JP-A- 2002 102 230 | JP-A- 2002 102 230 |
| JP-A- 2002 172 106 | JP-A- 2003 144 437 |
| US-A- 5 090 415 | US-A1- 2003 030 809 |
| US-B1- 6 397 099 | |

## Description

### Technical Field

The present invention relates to a scattering medium measuring apparatus and measuring method for internal information of a scattering medium such as a living body.

### Background Art

Recently, attention has focused on light-based living body measurement due to the advantages of being non-destructive and non-invasive. In such a measuring method, a scattering medium such as a living body to be measured is exposed to light having a predetermined wavelength such as near-infrared light so that the light propagates through the inside thereof. Then, a photodetector detects light emitted outside after propagation to obtain internal information (e.g. information about the concentration of oxygenated/deoxygenated hemoglobins in the living body) of the scattering medium from the detection result.

Various measurements may also be made such as providing multiple channels for such a scattering medium measurement to make a simultaneous multipoint measurement and thereby to obtain image data of the scattering medium. Such a technique for a scattering medium measurement using near-infrared light can be utilized for a brain function measurement in a living body, for example. A light-based measurement capable of reducing the size of an apparatus, achieving a low restriction, or obtaining a high sensitivity is suitable for measuring the activation of a brain easily (refer to Patent Documents 1 and 2 and Non-Patent Document 1, for example).

Patent Document 1: Japanese Patent Application Laid-Open No. Hei-09-184800
Patent Document 2: Japanese Patent Application Laid-Open No. 2001-178708
Non-Patent Document 1: H.Eda et al., "Multichannel time-resolved optical tomographic imaging system," Review of Scientific Instruments Vol.70, p.3595 (1999)

### Disclosure of the Invention

### Problems to be Solved by the Invention

As a method for measuring internal information of a scattering medium using near-infrared light, there has been known, for example, a method that uses CW light as measuring light, TRS (Time Resolved Spectroscopy) method that utilizes the time-resolved waveform of detected light for pulse light, or PMS (Phase Modulation Spectroscopy) method that utilizes modulated light. Among these methods, the TRS method allows the selection of an optical path length in a scattering medium by selecting the time component of detected light using the time-resolved waveform thereof, whereby it is possible to measure internal information accurately.

On the other hand, in such a light-based measurement, it is necessary to arrange an irradiation probe for irradiating a scattering medium with light and a detection probe for detecting light from the scattering medium at a constant spacing to form a channel for the scattering medium measurement. When trying to make a multichannel measurement using such an arrangement, there occurs a spatial restriction such as ensuring a sufficient distance between adjacent channels. Also, in the case of arranging adjacent channels closely to each other, there arises a problem of crosstalk between the channels.

JP-A-9 184800 shows a scattering medium measuring apparatus having first and second light emittiners one of which contains a dely means relative to the other. A single detector detects a signal transmitted through the scattering medium. EP-A-0,758,082, US-A-5.090,415, US-B-6,397,099 and US-A-2003/0030809 each also show scattering medium measuring apparatuses, having multiples light sources and detectors.

The present invention has been made to solve the above-described problems, and an object thereof is to provide a multichannel scattering medium measuring apparatus and measuring method capable of suppressing crosstalk between channels without a spatial restriction.

### Means for Solving the Problem

In order to achieve the foregoing object, the present invention is directed to a scattering medium measuring apparatus as set out in claim 1.

In the above-described scattering medium measuring apparatus, a plurality of measuring modules composed of one light irradiating means and a plurality of light detecting means are installed to achieve a multichannel measurement. Also, using pulse light and synchronizing the irradiation timing and detection timing thereof with each other allows for a TRS method-based measurement.

In addition, N pieces of measuring modules are adapted to irradiate and detect light at different timings. This allows crosstalk between adjacent channels to be suppressed even in the case of arranging measuring modules of the adjacent channels closely to each other. Also, since measuring modules are allowed to be arranged closely to each other, it is possible to make a measurement at a desired spatial resolution without a spatial restriction. Further, N pieces of measuring modules are used to irradiate and detect light successively to make a circuit of the measurement. This allows for a measurement of internal information of the scattering medium in a sufficient real-time manner in response to the change of internal information.

Here, the measuring apparatus comprises timing instruction means for instructing the light irradiating means and the light detecting means included in each of the N pieces of measuring modules, respectively, on the irradiation timing and the detection timing. In this case, the operation of the measuring apparatus can be controlled suitably. It may alternatively be arranged that the timing is controlled from an external apparatus.

Also, the interval of the irradiation timing between two of the light irradiating means having successive irradiation timings is preferably 1μsec or less. In this case, internal information can be measured in a sufficient real-time manner. In addition, the timing interval is further preferably 100nsec or less, for example, 50 to 60nsec.

With respect to the arrangement of the light irradiating means, it may be arranged that N pieces of light sources are installed to supply pulse light, respectively, to N pieces of the light irradiating means. It may alternatively be arranged that M pieces (M represents an integer of 1 or more to less than N) of light sources are installed to supply pulse light to a plurality of light irradiating means among N pieces of the light irradiating means.

## Effects of the Invention

The scattering medium measuring apparatus according to the present invention exhibits the following effects. That is, a plurality of measuring modules composed of one light irradiating means and a plurality of light detecting means are installed to achieve a multichannel measurement of the scattering medium. Also, using pulse light and synchronizing the irradiation timing and detection timing thereof with each other allows for the TRS method-based measurement.

In addition, N pieces of measuring modules are adapted to irradiate and detect light at different timings. This allows crosstalk between adjacent channels to be suppressed even in the case of arranging measuring modules of the adjacent channels closely to each other. Also, since measuring modules are allowed to be arranged closely to each other, it is possible to make a measurement at a desired spatial resolution without a spatial restriction. Further, N pieces of measuring modules are used to irradiate and detect light successively to make a circuit of the measurement. This allows for a measurement of internal information in a sufficient real-time manner in response to the change of internal information of the scattering medium.

## Brief Description of the Drawings

[FIG. 1] FIG. 1 is a block diagram showing the configuration of a scattering medium measuring apparatus according to a first embodiment.
[FIG. 2] FIG. 2 is a view schematically showing a scattering medium measuring method which uses the measuring apparatus shown in FIG. 1.
[FIG. 3] FIG. 3 is a timing chart showing the operation of the measuring apparatus shown in FIG. 1.
[FIG. 4] FIG. 4 is a view showing an example of arranging measuring modules in relation to a scattering medium.
[FIG. 5] FIG. 5 is a timing chart showing the operation of a measuring apparatus having the arrangement example shown in FIG. 4.
[FIG. 6] FIG. 6 is a view showing an example of arranging measuring modules in relation to a scattering medium.
[FIG. 7] FIG. 7 is a view showing an example of arranging measuring modules in relation to a scattering medium.
[FIG. 8] FIG. 8 is a graph showing time-resolved waveform data when varying the distance between a light irradiating position and a light detecting position.
[FIG. 9] FIG. 9 is a view showing the positional relationship between adjacent measuring modules.
[FIG. 10] FIG. 10 is a view showing the positional relationship between adjacent measuring modules.
[FIG. 11] FIG. 11 is a graph showing time-resolved waveform data based on light from an adjacent measuring module.
[FIG. 12] FIG. 12 is a graph showing the absorption spectrum of an oxygenated hemoglobin and deoxygenated hemoglobin.
[FIG. 13] FIG. 13 is a graph showing an example of a time-resolved waveform.
[FIG. 14] FIG. 14 is a view schematically showing the optical path distributions in a scattering medium corresponding to light components detected, respectively, at (a) $t = t_1$, (b) $t = t_2$, and (c) $t = t_3$.

[FIG. 15] FIG. 15 is a view showing a selection of depth information in a scattering medium based on a TRS method.
[FIG. 16] FIG. 16 is a block diagram showing the configuration of a scattering medium measuring apparatus according to a second embodiment.
[FIG. 17] FIG. 17 is a timing chart showing the operation of the measuring apparatus shown in FIG. 16.
[FIG. 18] FIG. 18 is a view showing an example of arranging measuring modules in relation to a scattering medium.
[FIG. 19] FIG. 19 is a block diagram showing an exemplary variation outside the scope of the present invention.
[FIG. 20] FIG. 20 is a block diagram showing an exemplary variation outside the scope of the present invention.
[FIG. 21] FIG. 21 is a view showing another example of arranging measuring modules in relation to a scattering medium, outside the scope of the present invention.

## Description of Symbols

1: First measuring module, 10: Pulse light source, 11: Irradiation probe, 60, 70: Photodetector, 61, 71: Detection probe, 61 a, 71a: Shutter, 62, 72: Signal processing circuit, 63, 73: A/D converter, 64, 74: Memory,
2: Second measuring module, 20: Pulse light source, 21: Irradiation probe, 22: Optical delay device, 80, 90: Photodetector, 81, 91: Detection probe, 81a, 91 a: Shutter, 82, 92: Signal processing circuit, 83, 93: A/D converter, 84, 94: Memory,
30: Pulse light source, 50: Trigger circuit, 51, 52: Delay circuit, 40: Photodetector, 41: Detection probe, 41a: Shutter, 42: Signal processing circuit, 43: A/D converter, 45: Selector, 46, 47: Memory.

## Best Modes for Carrying Out the Invention

Preferred embodiments of the scattering medium measuring apparatus and measuring method according to the present invention will hereinafter be described in detail with reference to the accompanying drawings. Additionally, in the descriptions of the drawings, identical components are designated by the same reference numerals to omit overlapping description. Also, the dimensional ratios in the drawings do not necessarily correspond to those in the descriptions.

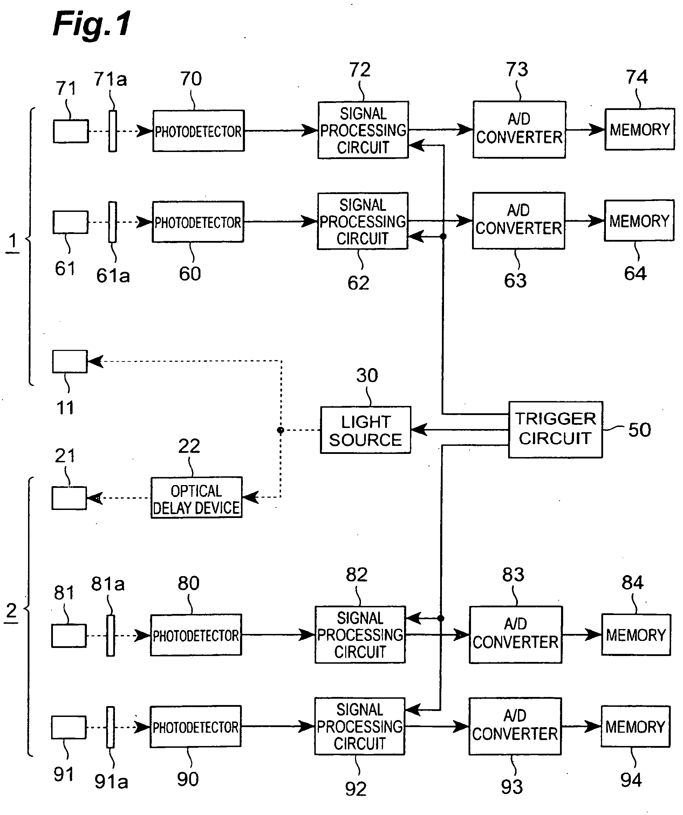

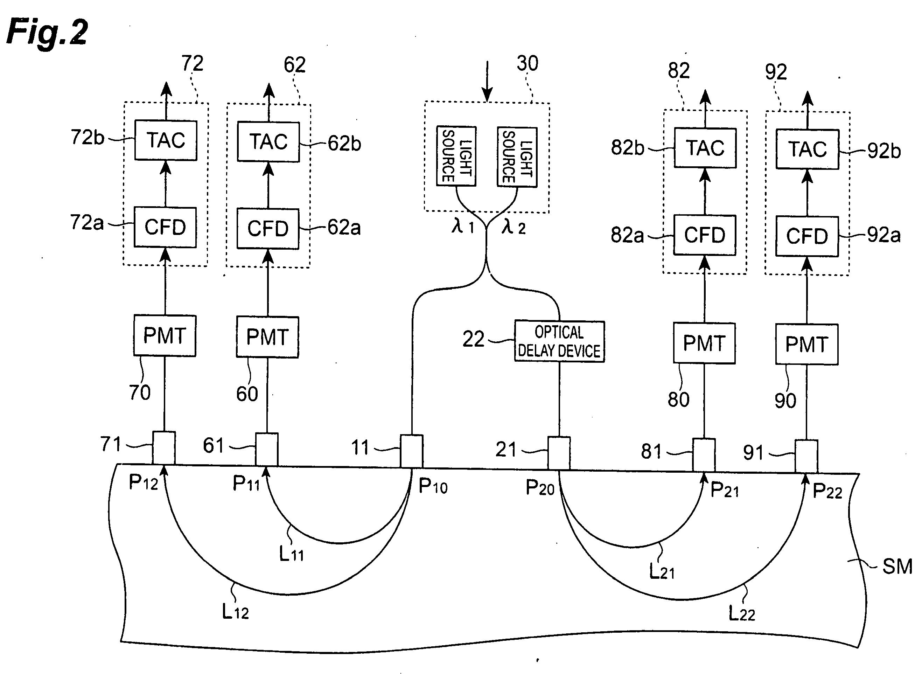

FIG. 1 is a block diagram schematically showing the configuration of a scattering medium measuring apparatus according to a first embodiment of the present invention. FIG. 2 is a view schematically showing a scattering medium measuring method which uses the scattering medium measuring apparatus shown in FIG. 1. The scattering medium measuring apparatus is adapted to measure internal information of a scattering medium non-invasively using the light-based TRS method such as near-infrared light. As a scattering medium to be measured using the measuring apparatus can be cited, for example, a living body. Also, as internal information to be measured can be cited, for example, the relative concentration of hemoglobins and oxygen saturation in a living body.

The measuring apparatus shown in FIG. 1 comprises two measuring modules, i.e., first and second measuring modules 1 and 2.

The first measuring module 1 has light irradiating means including an irradiation probe 11, first light detecting means including a first detection probe 61, and second light detecting means including a second detection probe 71. The irradiation probe 11 is arranged at a light irradiating position $P_{10}$ specified on a scattering medium SM (refer to FIG. 2). The irradiation probe 11 is connected with a pulse light source 30 for supplying pulse light (e.g. picosecond-order pulse light) to the scattering medium SM to measure internal information thereof non-invasively via an optical system such as an optical fiber, which constitutes the light irradiating means of the measuring module 1. Pulse light supplied from the light source 30 and having a predetermined wavelength passes through the optical fiber and the irradiation probe 11, and then irradiates the scattering medium SM from the light irradiating position $P_{10}$.

Here, in accordance with a specific measuring method, etc., as the pulse light source 30, used is a short pulse light source for supplying one wavelength of pulse light or a light source unit composed of a plurality of short pulse light sources for supplying pulse light having their respective different wavelengths as exemplified in FIG. 2.

The detection probe 61 is arranged at a first light detecting position $P_{11}$ specified on the scattering medium SM. The detection probe 61 is connected with a photodetector 60 for detecting light irradiated from the irradiation probe 11 and propagating through the inside of the scattering medium SM via an optical system such as an optical fiber, which constitutes the first light detecting means. Light made incident into the light detecting position $P_{11}$ of the scattering medium SM passes through the detection probe 61 and the optical fiber, and then the light intensity is to be detected in the photodetector 60. Also, between the detection probe 61 and the photodetector 60 is installed a shutter 61 a for controlling the passage of light from the detection probe 61 to the photodetector 60.

As is the case with the foregoing arrangement, the detection probe 71 is arranged at a second light detecting position $P_{12}$ specified on the scattering medium SM. The detection probe 71 is connected with a photodetector 70 via an optical fiber, etc., which constitutes the second light detecting means. Light made incident into the light detecting position $P_{12}$ of the scattering medium SM passes through the detection probe 71 and the optical fiber, and then the light intensity is to be detected in the photodetector 70. Also, between the detection probe 71 and the photodetector 70 is installed a shutter 71 a.

In addition, there are provided signal processing circuits 62 and 72, A/D converters 63 and 73, and memories 64 and 74 for detection signals outputted, respectively, from the photodetectors 60 and 70. These circuit systems are arranged in such a manner as to be capable of making a measurement using the time-correlated single-photon counting method in which discrete pulse light is detected and integrated.

More specifically, the signal processing circuits 62 and 72 are adapted to perform signal processing required to obtain intensity information or time information, etc., of detected light for detection signals from the photodetectors 60 and 70. The signal processing circuits 62 and 72 can be composed, respectively, of pulse height discriminators (CFDs) 62a and 72a for discriminating detection signals by input light from noise signals and time-amplitude converters (TACs) 62b and 72b for converting time information into voltage, as shown in FIG. 2, for example.

Also, the A/D converters 63 and 73 are adapted to convert electrical signals from the signal processing circuits 62 and 72 into digital signals. Then, digital signals output from the A/D converters 63 and 73 are stored, respectively, in histogram memories 64 and 74 to be used for generating time-resolved waveform of detected light and for obtaining internal information of the scattering medium SM using the waveform. Here, as the photodetectors 60 and 70, various photodetectors such as photomultiplier tubes (PMTs) exemplified in FIG. 2 and photodiodes can be used.

On the other hand, the second measuring module 2 has light irradiating means including an irradiation probe 21, first light detecting means including a first detection probe 81, and second light detecting means including a second detection probe 91. The irradiation probe 21 is arranged at a light irradiating position $P_{20}$ specified on a scattering medium SM. The irradiation probe 21 is connected with the pulse light source 30 via an optical fiber, etc., which constitutes the light irradiating means of the measuring module 2. That is, in the present embodiment, one pulse light source 30 is installed as a common light source for the two measuring modules 1 and 2, wherein pulse light supplied from the light source 30 is branched to be used in both the measuring modules 1 and 2.

Also, in the measuring module 2, between the light source 30 and the irradiation probe 21 is provided an optical delay device 22 for delaying pulse light supplied from the light source 30 by a predetermined time $\Delta T$. Pulse light supplied from the light source 30 and having a predetermined wavelength passes through the optical fiber, the optical delay device 22, and the irradiation probe 21, and then irradiates the scattering medium SM from the light irradiating position $P_{20}$. As the optical delay device 22, for example, an optical fiber having a predetermined length (optical path length) can be used.

The detection probe 81 is arranged at a first light detecting position $P_{21}$ specified on the scattering medium SM. The detection probe 81 is connected with a photodetector 80 via an optical fiber, etc., which constitutes the first light detecting means. Light made incident into the light detecting position $P_{21}$ of the scattering medium SM passes through the detection probe 81 and the optical fiber, and then the light intensity is to be detected in the photodetector 80. Also, between the detection probe 81 and the photodetector 80 is installed a shutter 81a.

As is the case with the foregoing arrangement, the detection probe 91 is arranged at a second light detecting position $P_{22}$ specified on the scattering medium SM. The detection probe 91 is connected with a photodetector 90 via an optical fiber, etc., which constitutes the second light detecting means. Light made incident into the light detecting position $P_{22}$ of the scattering medium SM passes through the detection probe 91 and the optical fiber, and then the light intensity is to be detected in the photodetector 90. Also, between the detection probe 91 and the photodetector 90 is installed a shutter 91 a.

In addition, there are provided signal processing circuits 82 and 92, A/D converters 83 and 93, and memories 84 and 94 for detection signals output, respectively, from the photodetectors 80 and 90. The arrangement of these circuit systems in the measuring module 2 is the same as that of the circuit systems in the measuring module 1.

There is installed a trigger circuit 50 for controlling the operation timing of each part of the measuring apparatus that comprises the above-described measuring modules 1 and 2. The trigger circuit 50 is a timing instruction means for instructing the light irradiating means and the light detecting means included in the measuring modules 1 and 2, respectively, on the irradiation timing and the detection timing of pulse light.

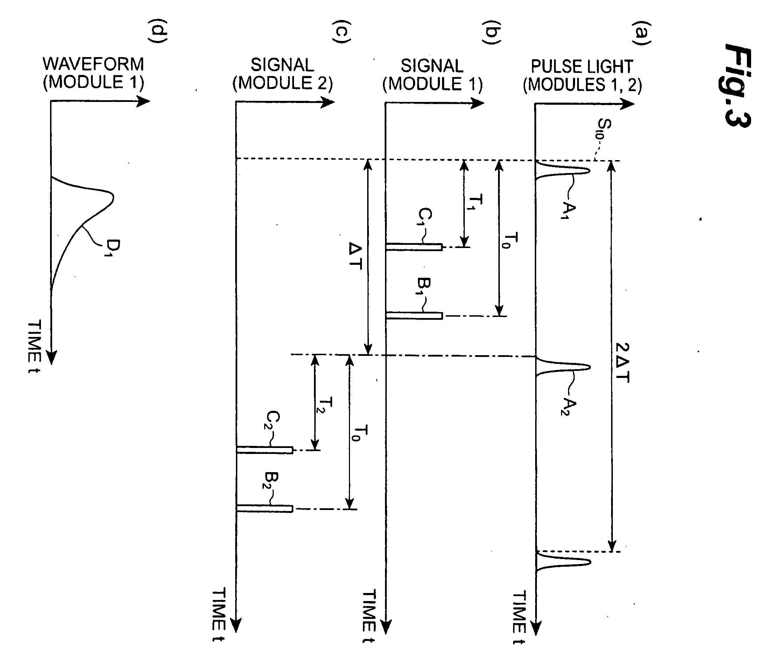

FIG. 3 is a timing chart showing the operation of the scattering medium measuring apparatus shown in FIG. 1. Here, in the timing chart of FIG. 3, the graph (a) shows pulse light applied in the measuring modules 1 and 2, the graph (b) shows a detection signal and a trigger signal input into a signal processing circuit in the measuring module 1, and the graph (c) shows a detection signal and a trigger signal input into a signal processing circuit in the measuring module 2.

In the present embodiment, the trigger circuit 50 is adapted to send a trigger signal for instructing one pulse light source 30 corresponding to the irradiation timing of pulse light from each of the irradiation probes 11 and 21 in the respective measuring modules 1 and 2, as indicated by the dashed line $S_{t0}$ in the graphs (a) to (c) of FIG. 3.

Pulse light emitted from the light source 30 in response to the trigger signal is to be applied from the irradiation probe 11 at a predetermined irradiation timing to the scattering medium SM as pulse light $A_1$ without passing through an optical delay device in the measuring module 1. Additionally, the pulse light is to be applied from the irradiation probe 21 to the scattering medium SM as pulse light $A_2$ after being delayed by a predetermined time $\Delta T$ in the optical delay device 22 in the measuring module 2 (graph (a)). This allows pulse light to be applied from the irradiation probes 11 and 21 corresponding to the two respective measuring modules 1 and 2 to the scattering medium SM successively at different irradiation timings of a timing interval $\Delta T$.

After the pulse light $A_1$ is irradiated from the irradiation probe 11 in the first measuring module 1, a light component passing through an optical path $L_{11}$ (refer to FIG. 2) reaches the detection probe 61 to be detected by the photodetector 60. The detection signal detected by the photodetector 60 is sent to the CFD 62a, and the CFD 62a outputs a detection signal $C_1$ delayed by a time $T_1$ from the irradiation timing of the pulse light $A_1$ (graph (b)). The detection of a light component passing through an optical path $L_{12}$ will be performed in the same manner as above by the photodetector 70.

On the other hand, the trigger circuit 50 is adapted to send a trigger signal for detecting light at a predetermined detection timing synchronized with the irradiation timing of the pulse light $A_1$ from the irradiation probe 11 to the two signal processing circuits 62 and 72 included in the measuring module 1. For example, in the case where the detection signal $C_1$ from the CFD 62a is input to the TAC 62b as a start signal, the trigger circuit 50 sends a trigger signal $B_1$ delayed by a time $T_0$ from the irradiation timing of the pulse light $A_1$ to be used as a stop signal in the TAC 62b (graph (b)). This allows a time-resolved measurement of the detection signal from the photodetector 60. The time-resolved measurement of a detection signal input from the photodetector 70 to the signal processing circuit 72 will be made in the same manner as above.

Subsequently, after the pulse light $A_2$ is irradiated from the irradiation probe 21 in the second measuring module 2, a light component passing through an optical path $L_{21}$ reaches the detection probe 81 to be detected by the photodetector 80. The detection signal detected by the photodetector 80 is sent to the CFD 82a, and the CFD 82a outputs a detection signal $C_2$ delayed by a time $T_2$ from the irradiation timing of the pulse light $A_2$ (graph (c)). The detection of a light component passing through an optical path $L_{22}$ will be performed in the same manner as above by the photodetector 90.

On the other hand, the trigger circuit 50 is adapted to send a trigger signal, e.g. trigger signal $B_2$ to be used as a stop signal in the TAC, for detecting light at a predetermined detection timing synchronized with the irradiation timing of the pulse light $A_2$ from the irradiation probe 21 to the two signal processing circuits 82 and 92 included in the measuring module 2 (graph (c)). This allows a time-resolved measurement of the detection signals from the photodetectors 80 and 90.

This completely goes once through a measurement of irradiating and detecting light successively using the two measuring modules 1 and 2. Then, repeating such a measurement multiple times and integrating the detection results thereof allows the time-resolved waveform of detected light to be used for a TRS method-based measurement of internal information of a scattering medium to be obtained. In the graph (d) of FIG. 3 is exemplarily shown a time-resolved waveform $D_1$ as an integration of time-voltage converted signals obtained by the detection signal $C_1$ and the trigger signal $B_1$ in the measuring module 1 shown in the graph (b). Additionally, the trigger circuit 50 may control not only the signal processing circuits but also, for example, the operation timing of shutters provided between the detection probes and the photodetectors. Also, in the case of using a photodetector having a gating function, the trigger circuit 50 may control the timing of a gating operation for switching the photodetector ON and OFF instead of the shutter.

In addition, although the number of measuring modules in FIG. 1 to FIG. 3 is two for simplification purposes, it is generally possible to construct a measuring apparatus comprising N pieces (N represents an integer of 2 or more) of measuring modules. Also, the number of light detecting means (detection probes) included in each measuring module may be generally at least one, that is, one or more.

The effect of the scattering medium measuring apparatus and measuring method according to the above-described embodiment will be described below.

In the measuring apparatus and measuring method shown in FIG. 1 to FIG. 3, a plurality of measuring modules composed of light irradiating means including an irradiation probe and light detecting means including a detection probe are installed to achieve a multichannel scattering medium measurement that allows image data to be obtained. Also, using not CW light but pulse light as measuring light and synchronizing the irradiation timing of pulse light in the light irradiating means and the detection timing of corresponding light in the light detecting means with each other allows for a TRS method-based measurement.

In addition, the two measuring modules 1 and 2 are adapted to irradiate and detect light at different timings. This allows crosstalk between adjacent channels to be suppressed even in the case of arranging the measuring modules 1 and 2 of the adjacent channels closely to each other on a scattering medium SM. Further, since measuring modules are allowed to be arranged closely to each other due to the suppression of crosstalk, it is possible to make a measurement at a desired spatial resolution without a spatial restriction.

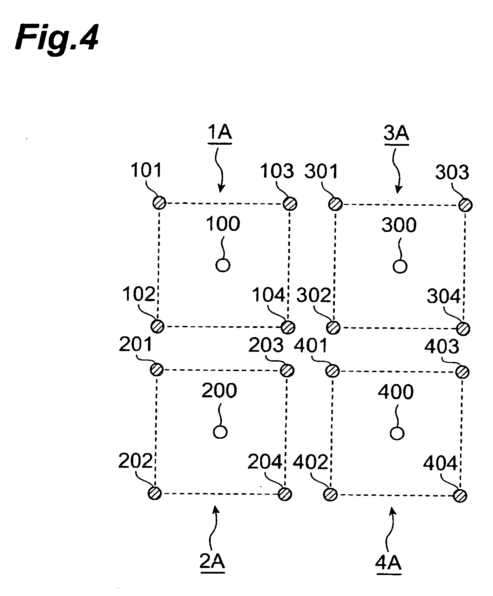

FIG. 4 is a view showing an example of arranging measuring modules in relation to a scattering medium. This figure shows an arrangement example in which four measuring modules 1A to 4A are arranged closely to each other. Among these measuring modules 1 A to 4A, the upper-left measuring module 1A is arranged to include an irradiation probe 100 and four detection probes 101 to 104 arranged at each vertex of a square centering on the irradiation probe 100. Also, the lower-left measuring module 2A, upper-right measuring module 3A, and lower-right measuring module 4A have the same arrangement as above.

If there is a time variation in internal information in a living body measurement, etc., each measuring module preferably irradiates and detects light simultaneously. However, in the arrangement shown in FIG. 4, in the case of irradiating pulse light simultaneously from each irradiation probe of the measuring modules 1A to 4A, for example, the detection probes 201 and 203 among those included in the measuring module 2A, which are positioned on the side of the measuring module 1A, receive not only the light from the irradiation probe 200 of the measuring module 2A but also the light from the irradiation probe 100 of the adjacent measuring module 1A, resulting in crosstalk between the channels.

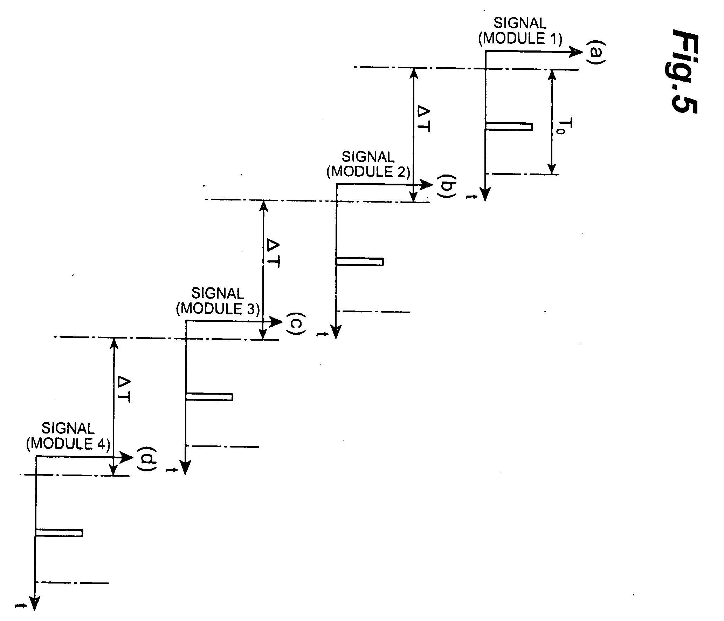

On the contrary, irradiating and detecting light successively at different timings as mentioned above allows for a measurement while preventing crosstalk between adjacent channels as shown in the graphs (a) to (d) of FIG. 5, which are timing charts of the operation for the arrangement example shown in FIG. 4.

Also, the measuring apparatus and measuring method according to the above-described embodiment goes once through a measurement of irradiating and detecting light successively using N pieces (2 pieces in the example shown in FIG. 1, while 4 pieces in the example shown in FIG. 4) of measuring modules, and then repeats the measurement multiple times to make a time-resolved measurement of detected light.

For example, as a method for obtaining such an integrated time-resolved waveform of detected light as shown in the graph (d) of FIG. 3 one of making a measurement multiple times successively using a measuring module to obtain an integrated time-resolved waveform, and of making such a measurement sequentially in each measuring module (refer to Non-Patent Document 1: H.Eda et al., Review of Scientific Instruments Vol.70, p.3595 (1999) for example) can be considered. However, in such a method, it is necessary to make a measurement multiple times in one measuring module to obtain a time-resolved waveform, resulting in a significant difference in the measurement timing for each measuring module (e.g. one to several seconds). Such a difference in measurement timing becomes a problem especially in the case of, for example, measuring the temporal change of oxygenated/deoxygenated hemoglobins in a living body.

On the contrary, switching a plurality of measuring modules one by one to make a measurement successively suppresses the difference in measurement timing between measuring modules down to about the interval $\Delta T$ of the irradiation timing of pulse light. This allows for a measurement of internal information of the scattering medium in a sufficient real-time manner in response to the change of internal information.

With respect to the measurement timing, the interval $\Delta T$ of the irradiation timing between two light irradiating means (two measuring modules) having successive irradiation timings of pulse light is preferably 1$\mu$sec or less. This allows for a measurement of internal information in a sufficient real-time manner in response to the measurement level of the scattering medium such as living body measurement level (about 100msec or more). In addition, the timing interval $\Delta T$ is further preferably 100nsec or less, for example, 50 to 60nsec.

Also, in the arrangement shown in FIG. 1, in the measuring apparatus is provided a trigger circuit 50 as timing instruction means for instructing the measuring modules 1 and 2 on the irradiation timing and the detection timing of light. This allows the operation of the measuring apparatus to be controlled suitably. However, with respect to such a timing instruction, it may alternatively be arranged that the timing is controlled from an external apparatus.

Here, in Patent Document 2: Japanese Patent Application Laid-Open No. 2001-178708 describes the art of arranging a plurality of light irradiation devices and light detection devices on a test body. However, the apparatus is arranged simply in such a manner that the light irradiation devices and the light detection devices are arranged alternately, but not composed of a plurality of measuring modules. On the contrary, in the measuring apparatus shown in FIG. 1, there are installed a plurality of measuring modules composed of light irradiating means and light detecting means in a corresponding manner, in each measuring module is made a time-resolved measurement with the irradiating and the detecting of light being synchronized with each other, and there is performed a successive measurement between a plurality of measuring modules. This can achieve a multichannel measuring apparatus capable of suppressing crosstalk between channels without a spatial restriction as mentioned above.

Also, with respect to the arrangement of a measuring apparatus comprising a plurality of measuring modules, the above-described arrangement can generally be applied to a measuring apparatus comprising N pieces (N represents an integer of 2 or more) of measuring modules with each having light irradiating means and at least one light detecting means as mentioned above. Further, the measuring modules may be arranged variously.

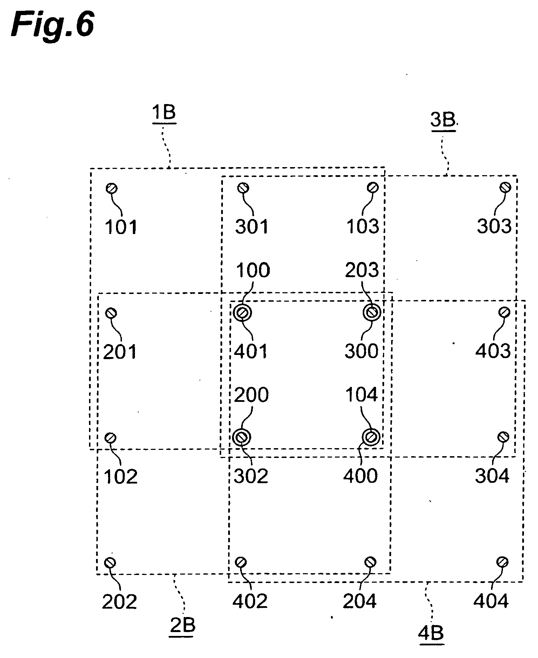

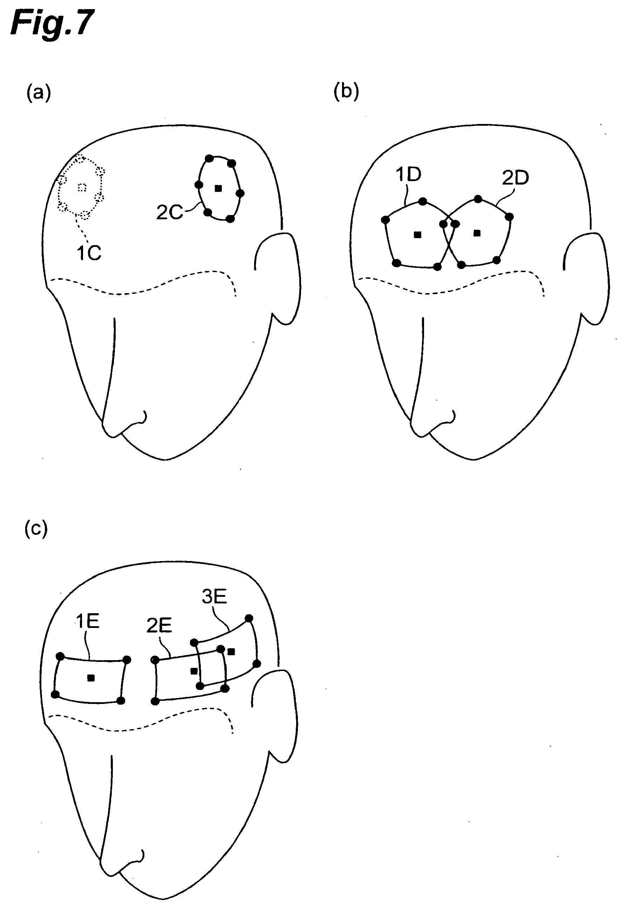

FIG. 6 and FIG. 7 are views showing other examples of arranging measuring modules in relation to a scattering medium. In the arrangement example shown in FIG. 6, four measuring modules 1B to 4B having the same configuration as that of the measuring modules 1A to 4A shown in FIG. 4 are arranged in such a manner as to be overlapped partially with each other. In this case, the distance between adjacent light detecting positions is approximately half of the case shown in FIG. 4. Thus, in accordance with the measuring apparatus and measuring method according to the foregoing arrangement, irradiation probes and detection probes can be arranged at a higher density in relation to a scattering medium to make a measurement at a higher resolution.

In the arrangement examples (a) to (c) of FIG. 7 are shown examples of arranging measuring modules when designating the head of a living body as a scattering medium to be measured. In each arrangement example, at the center of each measuring module is positioned an irradiation probe with a plurality of detection probes arranged therearound.

In the arrangement example (a) of FIG. 7, two measuring modules 1C and 2C with probes arranged in a regular hexagonal shape are arranged at a sufficient mutual distance so as not to have a problem with crosstalk. Also, in the arrangement example (b), two measuring modules 1D and 2D with probes arranged in a regular pentagonal shape are arranged in such a manner as to be overlapped partially with each other. Further, in the arrangement example (c), three measuring modules 1E, 2E and 3E with probes arranged in a rectangular shape are arranged closely to each other or in such a manner as to be overlapped partially with each other.

Thus, in the scattering medium measuring apparatus according to the present invention, measuring modules may be arranged variously. However, with respect to the arrangement in each measuring module, it is preferable to arrange probes in a shape where each light detecting position has the same distance from a light irradiating position such as a regular polygonal shape.

Additionally, in the case where an irradiation probe and a detection probe are extremely close to each other or in the same position, it is preferable to block light using, for example, a mechanical or liquid crystalline shutter to protect the photodetector connected to the detection probe. Also, in the case of using a photodetector having a gating function, the trigger circuit 50 may preferably control the timing of a gating operation for switching the photodetector ON and OFF. In this case, it is only required to perform a gating operation of stopping the operation of the photodetector while irradiating light from an irradiation probe.

Here, in the case of a PMT, gating means switching the PMT electrically to measure only a signal with a desired time width. Using such a gating function allows a photodetector to be protected from excessive light made incident instead of a shutter. Also, as a highspeed photodetector, a streak camera may be used. Further, as an irradiation probe and a detection probe, a bundle fiber in which a plurality of detecting optical fibers are arranged around an irradiating optical fiber may be used.

The effect of suppressing crosstalk between adjacent channels in accordance with the measuring apparatus and measuring method according to the foregoing arrangement will further be described below.

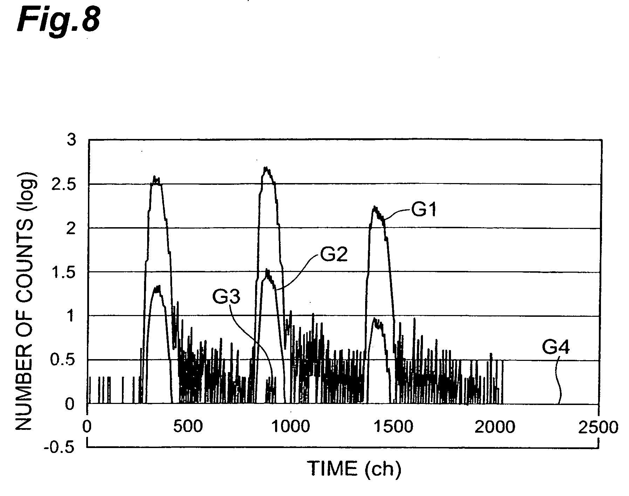

FIG. 8 is a graph showing time-resolved waveform data when varying the distance between a light irradiating position and a light detecting position. In this graph, the horizontal axis represents time (channel), while the vertical axis represents the number of counts (log). Also, the graphs G 1, G2, G3 and G4 show waveform data, respectively, at a distance of 4, 6, 8 and 10cm between a light irradiating position and a light detecting position. Additionally, 2000ch corresponds to about 50nsec for the horizontal axis.

For the waveform data, three types of short pulse laser beams having a wavelength of 760, 800 and 830nm are used as pulse light for irradiating a scattering medium. Correspondingly, in each graph shown in FIG. 8, a time-resolved waveform having three peaks corresponding to the three wavelengths is obtained. Also, the greater the distance between a light irradiating position and a light detecting position, the lower the light intensity and thereby peak height becomes lower, and the weighted center of the peaks deviate backward due to time delay.

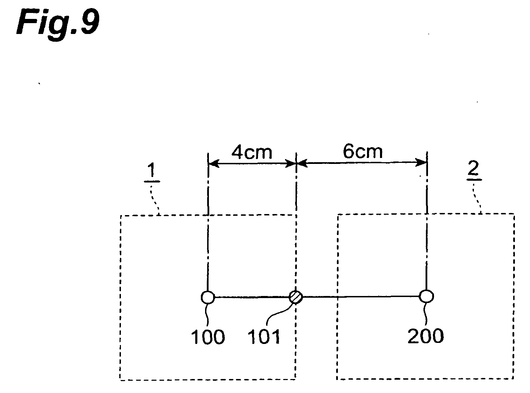

The distance between a light irradiating position and a light detecting position in an actual measurement is about 2.5 to 4cm. Assuming the distance between an irradiation probe 100 and a detection probe 101 in a measuring module 1 as 4cm, while the distance between an irradiation probe 200 in an adjacent measuring module 2 and the detection probe 101 as 6cm as shown in the arrangement example of FIG. 9, when irradiating pulse light simultaneously from the irradiation probes 100 and 200, the effect of crosstalk between the adjacent measuring modules is not small as found from the graphs G1 and G2 in FIG. 8.

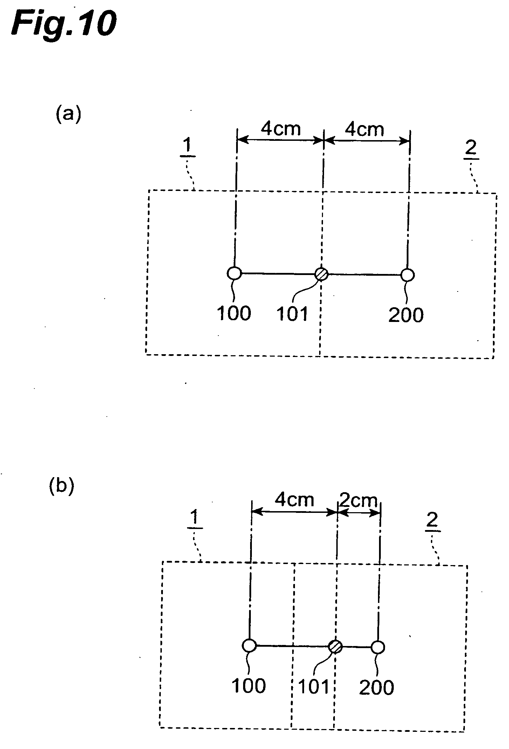

On the contrary, assuming the distance between the irradiation probe 200 in the adjacent measuring module 2 and the detection probe 101 as 8cm or more reduces the effect of crosstalk, while the measuring modules cannot be arranged closely to each other, resulting in significant restriction in a measurement. Further, in the case where adjacent measuring modules 1 and 2 are in contact with each other as shown in the arrangement example (a) of FIG. 10 or where adjacent measuring modules 1 and 2 are arranged in such a manner as to be overlapped partially with each other as shown in the arrangement example (b) of FIG. 10, it is impossible to make an accurate measurement.

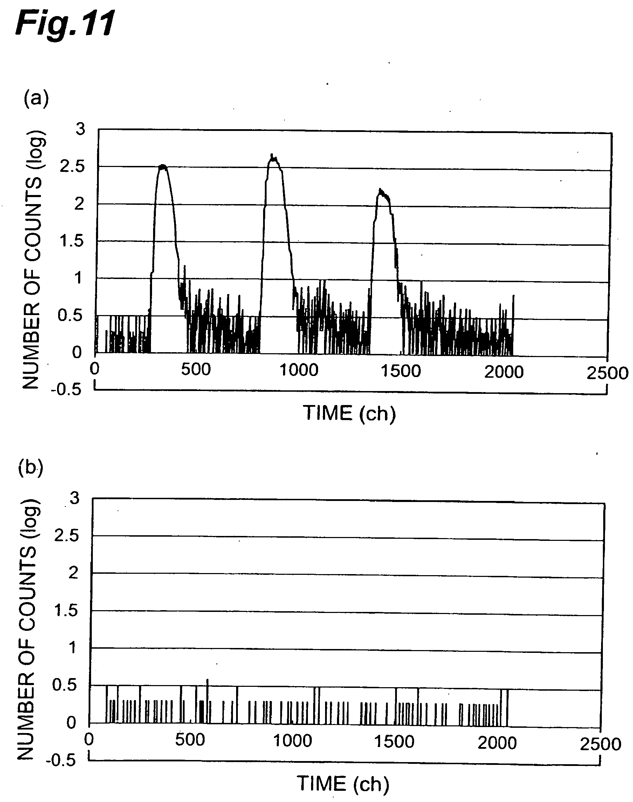

For example, in the arrangement example (a) of FIG. 10, the detection probe 101 measures light from the irradiation probe 200 that is 4cm apart therefrom as shown in the graph (a) of FIG. 11. On the contrary, in the arrangement example (a) of FIG. 10, in accordance with the above-described method that uses a plurality of measuring modules for a successive synchronized measurement, the detection probe 101 does not measure light from the irradiation probe 200 as shown in the graph (b) of FIG. 11. Thus appropriately specifying the interval of measurement timing between a plurality of measuring modules (e.g. 50nsec) to make a successive measurement allows crosstalk between channels to be suppressed without a spatial restriction in respect to, for example, the arrangement of probes.

Next will be described the TRS method-based measurement of internal information of a scattering medium to be used in the above-described measuring apparatus and measuring method as well as the advantages thereof in the case of measuring the concentration of oxygenated hemoglobins ($HbO_2$) and deoxygenated hemoglobins (Hb) in a living body, for example.

Although oxygen in a living body cannot be measured directly in a light-based living body measurement, the light absorption spectrum of pigment protein such as hemoglobin, which is involved in oxygen metabolism in blood, differs between an oxygenated state and a deoxygenated state, whereby it is possible to indirectly obtain information about oxygen metabolism in a living body.

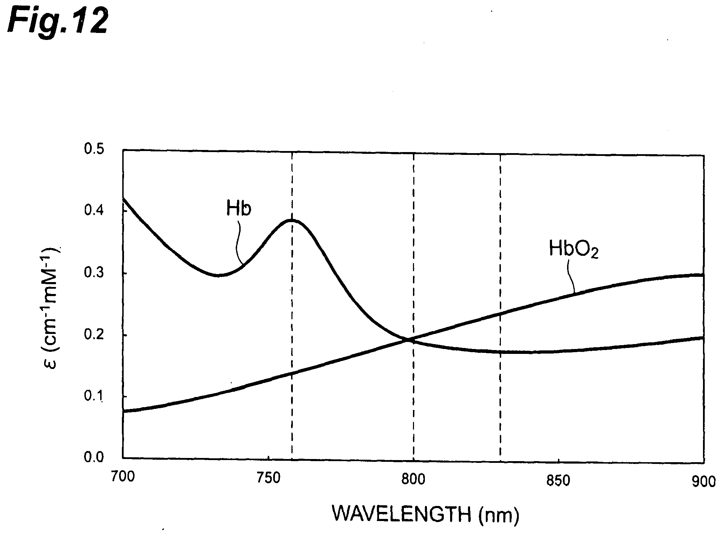

The absorption spectrums (wavelength dependency of optical absorption property) of oxygenated hemoglobins and deoxygenated hemoglobins are shown in FIG. 12. In the absorption spectrum of hemoglobins, although light is attenuated significantly in a wavelength range of 600nm or less to make it difficult to measure transmitted light, the use of near-infrared light (having a wavelength of, for example, 700 to 1100nm which can be less attenuated in a living body) allows for such a transmitted light measurement.

The absorbance A for near-infrared light that transmits through a living tissue can be represented approximately by the following Equation (1) by applying the Beer-Lambert law:

[Equation 1]

$$A = \log(I_0 / I)$$
$$= \left\{ \varepsilon_{HbO_2}[HbO_2] + \varepsilon_{Hb}[Hb] \right\} L + S \qquad \cdots (1)$$

Here, $I_0$ represents the amount of light incident, I the amount of transmitted light, $\varepsilon_{EHbO2}$ the absorbance coefficient of $HbO_2$, $\varepsilon_{Hb}$ the absorbance coefficient of Hb, $[HbO_2]$ the concentration of oxygenated hemoglobins, [Hb] the concentration of deoxygenated hemoglobins, L the effective optical path length, and S the extinction degree due to scattering. In the case of actually calculating the concentration of hemoglobins using Equation (1), some lights having a wavelength that exists in the near-infrared light range are used. In FIG. 12 are shown three wavelengths of 760, 800 and 830nm as an example.

In such a light-based measurement, the measuring method that uses CW light as measuring light has a problem of running short of information regarding the optical path length of light to be detected in a scattering medium to make a quantitative measurement. Meanwhile, the TRS method-based measuring method that uses pulse light exhibits an advantage that a variety of information including information regarding the optical path length can be obtained.

The behavior of light in a scattering medium such as a living body can be described by a light-diffusion equation as a function of the scattering coefficient and the absorbance coefficient for the light and the distance between a light irradiating position and a light detecting position. For example, when pulse light sufficiently short in time has entered a half-infinite medium, the light intensity R (p, t) at a time "t" at a position "r" on the same plane as the incident point can be represented by the following Equation (2):

[Equation 2]

$$R(\rho, t) = (4\pi Dc)^{-3/2} z_0 t^{-5/2} \exp(-\mu_a ct) \exp\left(-\frac{\rho^2 + z_0^2}{4Dc}\right) \qquad \cdots (2)$$

Here, p (cm) represents the position of a photodetector, t (sec) the time after the pulse light made incident, D (cm) the diffusion coefficient $D = 1/3\mu_s'$, $\mu_s'$ (cm$^{-1}$) the equivalent scattering coefficient, c (cm/sec) the light speed in the medium, $z_0$ (cm) the value $z_0 = 1/\mu_s'$, and $\mu_a$ (cm$^{-1}$) the absorbance coefficient. The absorbance coefficient and the scattering coefficient for light can be calculated by using the light-diffusion equation to analyze a time-resolved waveform obtained by a TRS method-based scattering medium measurement that uses pulse light. Then, utilizing the relationship between the absorbance coefficient and the concentration of absorbing substance (e.g. hemoglobin) allows for a quantitative determination of the concentration of the absorbing substance in the scattering medium.

In optical mapping and optical CT using such a scattering medium measuring method, it is one of the goals, for example, to obtain a two- or three-dimensional distribution of the oxygen concentration in a living body. Optical CT has gained attention as a simple and safe imaging diagnostic method capable of making a successive measurement non-invasively relative to PET and fMRI.

Although in order to perform an image reconstruction through optical mapping (including two- or three-dimensional) or optical CT, it is necessary to obtain sufficient internal information about the living body, and the CW light-based measurement can only obtain a limited amount of information. On the contrary, the TRS method-based measurement can obtain extremely large amounts of information through waveform data composed of, for example, 500 pieces of data even in the case of a single pair of a light source and photodetector, which is advantageous for image reconstruction, etc.

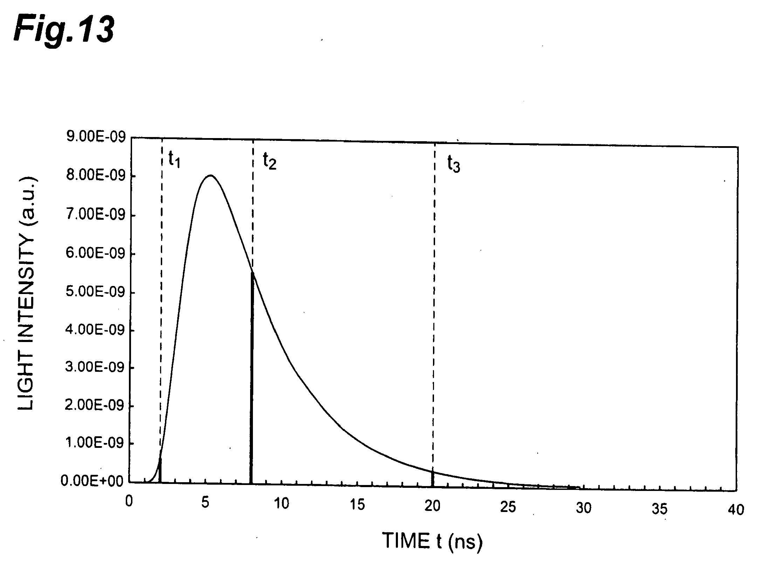

FIG. 13 is a graph showing an example of time-resolved waveform obtained through a TRS method-based scattering medium measurement. In this graph, the horizontal axis represents time t (ns), while the vertical axis represents the light intensity detected (a.u.). In such waveform data, here will be considered the case of selecting the time components at t = $t_1$ = 2ns, $t_2$ = 8ns, and $t_3$ = 20ns.

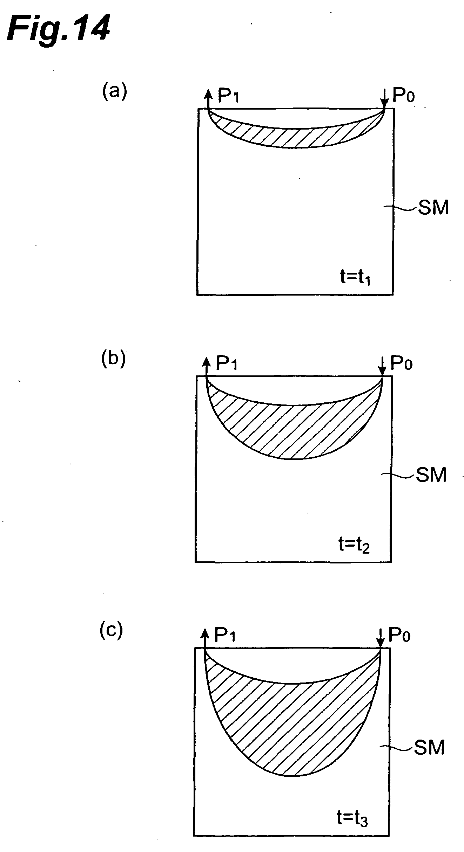

FIG. 14 is a view schematically showing the optical path distributions in a scattering medium SM corresponding to light components detected, respectively, at (a) $t = t_1$, (b) $t = t_2$, and (c) $t = t_3$. Here, a two-dimensional planar model is used, 80mm on a side, with the assumption that the distance between a light irradiating position $P_0$ and a light detecting position $P_1$ is 75mm.

When the time-resolved waveform data shown in FIG. 13 is obtained between the light irradiating position $P_0$ and the light detecting position $P_1$, in the waveform data, the optical path distribution of the corresponding light component in the scattering medium SM varies in accordance with the detection time "t." More specifically, as shown in the optical path distributions (a) to (c) of FIG. 14, the longer the delay in the time "t," the longer the optical path length in the scattering medium SM, and thereby the deeper the optical path distribution becomes. Therefore, making a measurement using the TRS method and selecting a light component using the obtained time-resolved waveform data in accordance with the detection time "t" allows the selection of depth information in the scattering medium at the identical measuring positions $P_0$ and $P_1$.

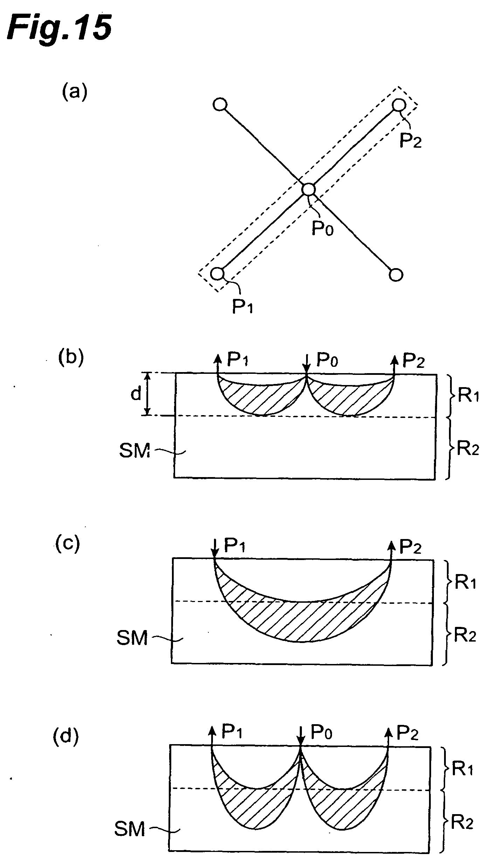

The selection of depth information in a scattering medium in the TRS method will further be described below with reference to FIG. 15. Here, the case of a measurement using three measuring positions $P_0$, $P_1$ and $P_2$ specified on a scattering medium for the probe arrangement shown in the arrangement example (a) of FIG. 15 will be considered. These measuring positions are arranged at the same spacing in the order of $P_1$, $P_0$ and $P_2$.

In such an arrangement as above, the region $R_1$ (first layer) from the surface with each measuring position specified thereon to a depth of "d" and the region $R_2$ (second layer) of a depth of "d" or more are specified as regions to obtain internal information of the scattering medium SM to be measured. Such a region is specified correspondingly to the layer structure from the epidermis to inside the body in, for example, a living body measurement.

In the case of thus specifying a plurality of measuring regions, since it is impossible to select depth information through a CW light-based measuring method, it is necessary to change the distance between the measuring positions as shown in the optical path distributions (b) and (c) of FIG. 15. That is, in the case of measuring the shallow region $R_1$, $P_0$ is specified as a light irradiating position, while $P_1$ and $P_2$ as light detecting positions to reduce the distance between the light irradiating position and each light detecting position. On the other hand, in the case of measuring the deep region $R_2$, $P_1$ is specified as a light irradiating position, while $P_2$ as a light detecting position to increase the distance between the light irradiating position and the light detecting position. In such a measurement, since it is necessary to switch a light irradiating position and a light detecting position, it is impossible to obtain data of the regions $R_1$ and $R_2$ simultaneously. It is also necessary to use a probe having both functions of light irradiating means and light detecting means such as a coaxial fiber for the measuring position $P_1$.

On the contrary, in a TRS method-based measuring method, it is possible to select depth information by selecting a light component in accordance with the detection time "t" as mentioned above. Therefore, as shown in the optical path distributions (b) and (d) of FIG. 15, the regions $R_1$ and $R_2$ can be measured simultaneously while keeping the same arrangement that $P_0$ is specified as a light irradiating position, while $P_1$ and $P_2$ as light detecting positions. Thus, in a TRS method-based scattering medium measurement, it is possible to obtain extremely large amounts of information effectively using time-resolved waveform data.

The scattering medium measuring apparatus and measuring method according to the present invention will further be described below.

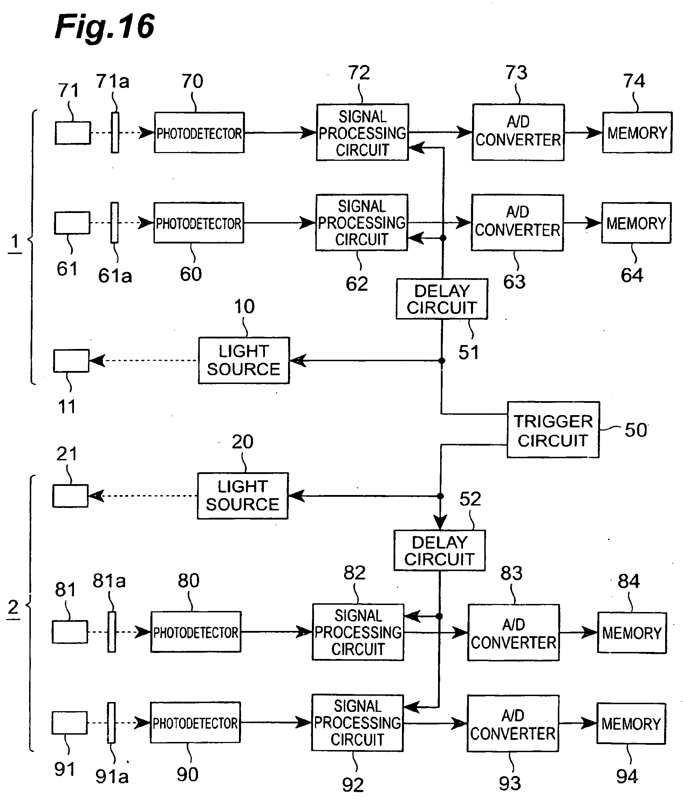

FIG. 16 is a block diagram schematically showing the configuration of a scattering medium measuring apparatus according to a second embodiment of the present invention. The measuring apparatus shown in FIG. 16 comprises two measuring modules, i.e., first and second measuring modules 1 and 2.

The first measuring module 1 has light irradiating means including an irradiation probe 11, first light detecting means including a first detection probe 61, and second light detecting means including a second detection probe 71. Among these components, the arrangement of the irradiation probe 11 in the light irradiating means, the detection probe 61, photodetector 60, and circuit systems in the first light detecting means, and the detection probe 71, photodetector 70, and circuit systems in the second light detecting means is the same as those shown in FIG. 1.

The irradiation probe 11 is connected with a pulse light source 10 for supplying pulse light to a scattering medium SM (refer to FIG. 2) to measure internal information thereof non-invasively via an optical system such as an optical fiber, which constitutes the light irradiating means of the measuring module 1. Pulse light supplied from the light source 10 and having a predetermined wavelength passes through the optical fiber and the irradiation probe 11, and then irradiates the scattering medium SM from a light irradiating position $P_{10}$.

Meanwhile, the second measuring module 2 has light irradiating means including an irradiation probe 21, first light detecting means including a first detection probe 81, and second light detecting means including a second detection probe 91. Among these components, the arrangement of the irradiation probe 21 in the light irradiating means, the detection probe 81, photodetector 80, and circuit systems in the first light detecting means, and the detection probe 91, photodetector 90, and circuit systems in the second light detecting means is the same as those shown in FIG. 1.

The irradiation probe 21 is connected with a pulse light source 20 via an optical fiber, etc., which constitutes the light irradiating means of the measuring module 2. That is, in the present embodiment, two pulse light sources 10 and 20 are installed individually for each of the two measuring modules 1 and 2, and pulse light supplied from the light sources 10 and 20 are used, respectively, in the measuring modules 1 and 2. Pulse light supplied from the light source 20 and having a predetermined wavelength passes through the optical fiber and the irradiation probe 21, and then irradiates the scattering medium SM from a light irradiating position $P_{20}$.

There is installed a trigger circuit 50 for controlling the operation timing of each part of the measuring apparatus that comprises the above-described measuring modules 1 and 2. The trigger circuit 50 is a timing instruction means for instructing the light irradiating means and the light detecting means included in the measuring modules 1 and 2, respectively, on the irradiation timing and the detection timing of pulse light.

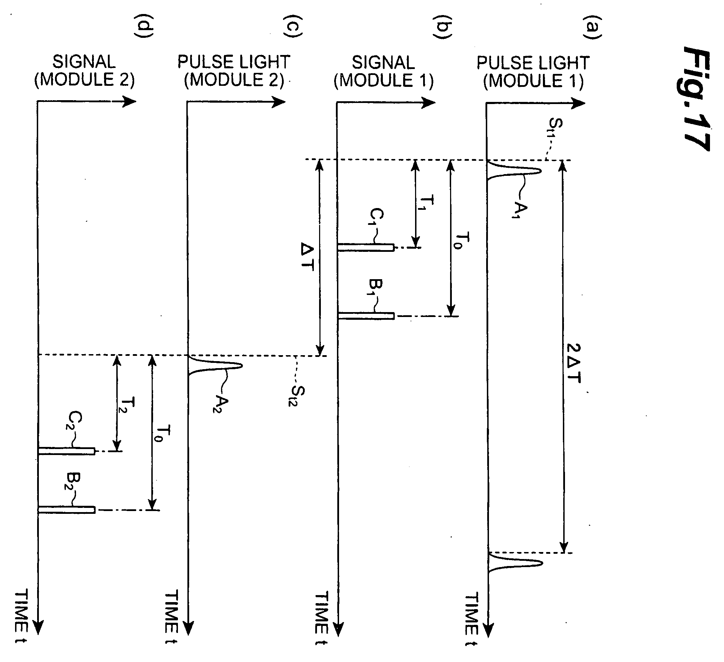

FIG. 17 is a timing chart showing the operation of the scattering medium measuring apparatus shown in FIG. 16. Here, in FIG. 17, the graph (a) shows pulse light applied in the measuring module 1, the graph (b) shows a detection signal and a trigger signal input into a signal processing circuit in the measuring module 1, the graph (c) shows pulse light applied in the measuring module 2, and the graph (d) shows a detection signal and a trigger signal input into a signal processing circuit in the measuring module 2.

In the present embodiment, the trigger circuit 50 is adapted to send trigger signals for instructing two pulse light sources 10 and 20 on the irradiation timing of pulse light from each of the irradiation probes 11 and 21 in the respective measuring modules 1 and 2, as indicated by the dashed line $S_{t1}$ in the graphs (a) and (b) and by the dashed line $S_{t2}$ in the graphs (c) and (d) of FIG. 17.

Pulse light emitted from the light sources 10 and 20 in response to the trigger signals is to be applied from the irradiation probes 11 and 21 at a predetermined irradiation timing to the scattering medium SM as pulse light $A_1$ and $A_2$ without passing through an optical delay device in both the measuring modules 1 and 2. Also, the trigger signal for the pulse light source 20 is delayed by a predetermined time $\Delta T$ from the trigger signal for the pulse light source 10 to be sent (graphs (a) and (c)). This allows pulse light to be applied from the irradiation probes 11 and 21 corresponding to the two respective measuring modules 1 and 2 to the scattering medium SM successively at different irradiation timings of an interval $\Delta T$.

After the pulse light $A_1$ is irradiated from the irradiation probe 11 in the first measuring module 1, a light component reaching the detection probe 61 is detected by the photodetector 60. The photodetector 60 outputs a detection signal $C_1$ delayed by a time $T_1$ from the irradiation timing of the pulse light $A_1$ (graph (b)) in response to the detected light. The detection of a light component by the photodetector 70 will be performed in the same manner as above.

On the other hand, the trigger signal for the light source 10 from the trigger circuit 50 is branched to be sent also to two signal processing circuits 62 and 72 included in the measuring module 1. The branched trigger signal enters the signal processing circuits 62 and 72 via a delay circuit 51 as a trigger signal, e.g. trigger signal $B_1$ to be used as a stop signal in the TAC, for detecting light at a predetermined detection timing synchronized with the irradiation timing of the pulse light $A_1$ from the irradiation probe 11 (graph (b)). This allows a time-resolved measurement of the detection signals from the photodetectors 60 and 70.

Subsequently, after the pulse light $A_2$ is irradiated from the irradiation probe 21 in the second measuring module 2, a light component reaching the detection probe 81 is detected by the photodetector 80. The photodetector 80 outputs a detection signal $C_2$ delayed by a time $T_2$ from the irradiation timing of the pulse light $A_2$ (graph (d)) in response to the detected light. The detection of a light component by the photodetector 90 will be performed in the same manner as above.

On the other hand, the trigger signal for the light source 20 from the trigger circuit 50 is branched to be sent also to two signal processing circuits 82 and 92 included in the measuring module 2. The branched trigger signal enters the signal processing circuits 82 and 92 through a delay circuit 52 as a trigger signal, e.g. trigger signal $B_2$ to be used as a stop signal in the TAC, for detecting light at a predetermined detection timing synchronized with the irradiation timing of the pulse light $A_2$ from the irradiation probe 21 (graph (d)). This allows a time-resolved measurement of the detection signals from the photodetectors 80 and 90.

This completely goes once through a measurement of irradiating and detecting light successively using the two measuring modules 1 and 2. Then, repeating such a measurement multiple times and integrating the detection results thereof allows the time-resolved waveform of detected light to be used for a TRS method-based measurement of internal information of a scattering medium to be obtained. Here, the trigger circuit 50 may control not only the signal processing circuits but also, for example, the operation timing of shutters provided between the detection probes and the photodetectors. Also, in the case of using a photodetector having a gating function, the trigger circuit 50 may control the timing of a gating operation for switching the photodetector ON and OFF instead of the shutter.

The effect of the scattering medium measuring apparatus and measuring method according to the above-described embodiment will be described below.

In the measuring apparatus and measuring method shown in FIG. 16 and FIG. 17, a plurality of measuring modules are installed to achieve a multichannel scattering medium measurement. Also, using pulse light and synchronizing the irradiation timing of pulse light and the detection timing of light with each other allows for a TRS method-based measurement.

In addition, the two measuring modules 1 and 2 are adapted to irradiate and detect light at different timings.

This allows crosstalk between adjacent channels to be suppressed even in the case of arranging the measuring modules 1 and 2 of the adjacent channels closely to each other on a scattering medium SM. Further, since measuring modules are allowed to be arranged closely to each other, it is possible to make a measurement at a desired spatial resolution without a spatial restriction.

Also, the measuring apparatus and measuring method according to the above-described embodiment goes once through a measurement of irradiating and detecting light successively using N pieces (2 pieces in the example shown in FIG. 16) of measuring modules, and then repeats the measurement multiple times to make a time-resolved measurement of detected light. Thus, switching a plurality of measuring modules one by one to make a measurement successively suppresses the difference in measurement timing between measuring modules down to about the interval ΔT of the irradiation timing of pulse light. This allows for a measurement of internal information of the scattering medium in a sufficient real-time manner in response to the change of internal information.

The scattering medium measuring apparatus and measuring method according to the present invention is not restricted to the above-described embodiments, and various modifications may be made. For example, the number of light detecting means (detection probes) to be provided in each measuring module may appropriately be one or more. The specific arrangement of irradiation probes and detection probes may also be changed variously.

Further, with respect to the arrangement of the light irradiating means for irradiating a scattering medium with pulse light, it is generally arranged, in such a configuration as shown in FIG. 16, that N pieces of light sources are installed to supply pulse light, respectively, to N pieces of the light irradiating means. Also, it is generally arranged, in such a configuration as shown in FIG. 1, that M pieces (M represents an integer of 1 or more to less than N) of light sources are installed to supply pulse light to a plurality of light irradiating means among N pieces of the light irradiating means. An optical switch, etc., may also be used for the switching of pulse light application.

In addition, with respect to the arrangement of the light detecting means, it is arranged, in the above-described embodiment, that all the light detecting means (detection probes) are included in one measuring module. On the contrary, it may be arranged that part of the light detecting means provided in each measuring module is shared by a plurality of measuring modules.

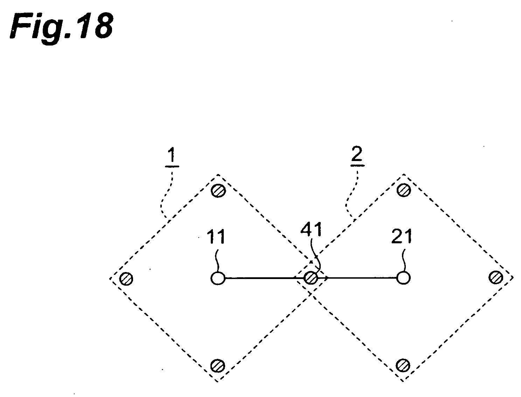

FIG. 18 shows such an example of arranging measuring modules.

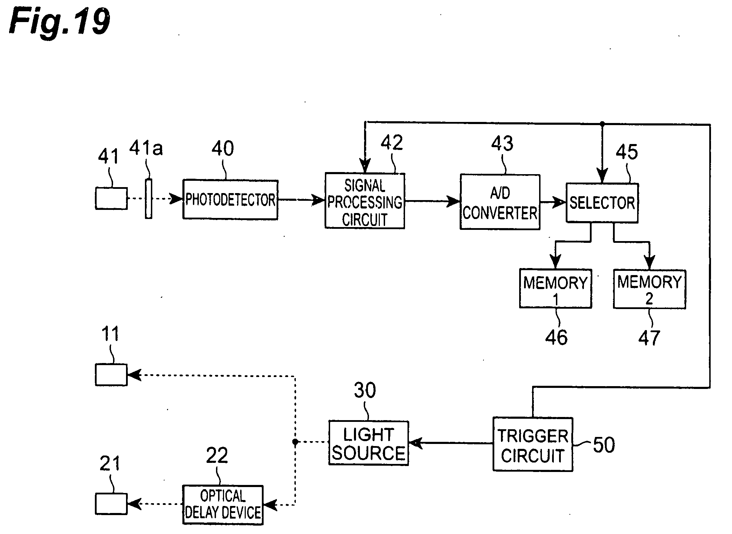

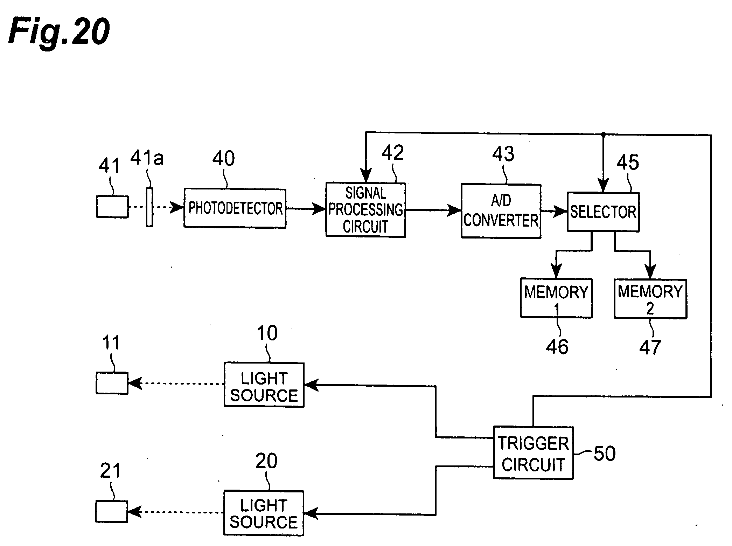

FIG. 19 is a block diagram showing an exemplary variation of the configuration of the measuring apparatus shown in FIG. 1 outside the scope of the present invention when using the probe arrangement shown in FIG. 18. FIG. 20 is also a block diagram showing an exemplary variation of the configuration of the measuring apparatus shown in FIG. 16 outside the scope of the present invention when using the probe arrangement shown in FIG. 18. Here, the components having the same configurations as those shown in FIG. 1 and FIG. 16 are omitted in FIG. 19 and FIG. 20.

In the arrangement examples above, in response to the arrangement that the detection probe 41 is used for the detection of light from both the irradiation probes 11 and 21, there are provided two memories, i.e., a first memory 46 to be used for a measurement in the measuring module 1 and a second memory 47 to be used for a measurement in the measuring module 2 as a histogram memory to be provided behind the shutter 41 a, photodetector 40, signal processing circuit 42, and A/D converter 43. Also, between the A/D converter 43 and the two memories 46 and 47 is provided a selector 45.

In such an arrangement, a trigger signal sent from the trigger circuit 50 to the signal processing circuit 42 for detection timing instruction of light enters the selector 45. The selector 45 sorts digital data from the A/D converter 43 into the memories 46 and 47 depending on which of the irradiation probes 11 and 21 irradiates the pulse light in accordance with an instruction signal from the trigger circuit 50. Accordingly, even if the detection probe 41 is shared by the measuring modules 1 and 2, it is possible to obtain data corresponding to the respective modules separately.

Thus, with respect to the arrangement of the light detecting means, it may be arranged variously including the arrangement that part of the light detecting means is shared by a plurality of measuring modules. Also, with the arrangement that a detection probe is shared and that one probe is used both as an irradiation probe and a detection probe, it is possible to increase the number of measurement data to be obtained.

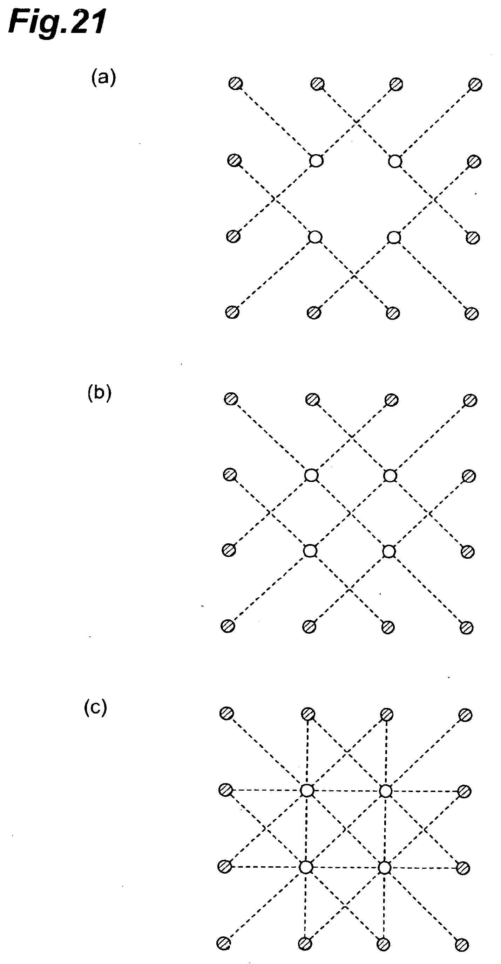

FIG. 21 is a view showing another example of arranging measuring modules in relation to a scattering medium outside the scope of the present invention. Here, in the arrangement examples (a) to (c) of FIG. 21, the dashed lines connecting probes indicate measuring points (data obtaining sections) at which measurement data can be obtained. Also, the distance between adjacent probes is 3cm. The arrangement examples (a) to (c) of FIG. 21 have the same arrangement that four irradiation probes (open circles) are arranged inside, while twelve detection probes (filled circles) are arranged outside, with the difference in conditions such as the sharing of detection probes.

In the arrangement example (a) of FIG. 21, the four inside probes are used only as irradiation probes, and the twelve outside detection probes are each related to one irradiation probe. In this case, the number of measuring points at which measurement data can be obtained is 12. Also, in the arrangement example (b), the four inside probes are used both as irradiation probes and detection probes. In this case, the number of measuring points is 14, which is greater than that in the arrangement example (a) by 2.

Further, in the arrangement example (c), the four inside probes are used both as irradiation probes and detection probes, and each detection probe is shared by a plurality of irradiation probes (measuring modules). In this case, the number of measuring points is 26, which is still greater than that in the arrangement example (b) by 12. Thus, the sharing and combinational use of probes increases the number of measurement data to be obtained, which meets the imaging conditions more suitably.

However, in the arrangement that the probes are used both as irradiation probes and detection probes as shown in the arrangement examples (b) and (c) of FIG. 21, since it is necessary to stop the function of the probes as detection probes when used as irradiation probes, it is preferable to use a PMT capable for switching the power supply ON and OFF at high speed. Also, in the case where each detection probe is shared by a plurality of measuring modules as shown in the arrangement example (c), in order to sort data at high speed, it is preferable to store data by switching memories, etc., sequentially not on a software level but on a hardware level.

**Industrial Applicability**

The present invention is available as a scattering medium measuring apparatus and measuring method capable of suppressing crosstalk between channels without a spatial restriction.

**Claims**

1. A scattering medium measuring apparatus for performing measurement using time resolved spectroscopy method comprising
   N pieces (N represents an integer of 2 or more) of measuring modules (1,2) with each consisting of one light irradiating means for irradiating a scattering medium (SM) with pulse light irradiated from a predetermined light irradiating position ($P_{10}$, $P_{20}$) to measure internal information thereof non-invasively, and a plurality of light detecting means for detecting light irradiated from the light irradiating means and propagating through the inside of the scattering medium (SM) at predetermined light detecting positions ($P_{11}$, $P_{12}$; $P_{21}$, $P_{22}$) **characterized by**:

   timing instruction means (50) for instructing the light irradiating means and the plurality of light detecting means included in each of the N pieces of measuring modules (1,2), respectively, on an irradiation timing and a detecting timing, wherein
   in each of the N pieces of measuring modules (1,2), an irradiation probe (60, 70; 22) of the light irradiating means is positioned a the center of the measuring module (1,2), detection probes (61a, 71a, 81a, 91a,; 41a) of the plurality of light detecting means are arranged around the irradiation probe (60, 70; 22) and the irradiation probe (60, 70; 22) and the detection probes (61a, 71a, 81a, 91a; 41a) are arranged so that each light detecting position ($P_{11}$, $P_{12}$; $P_{21}$, $P_{22}$) has the same distance from the light irradiating position ($P_{10}$, $P_{20}$) and wherein based on instruction signals from the timing instruction means (50) N pieces of the light irradiating means corresponding, respectively, to the N pieces of measuring modules (1,2) are adapted to irradiate the scattering medium (SM) with the pulse light successively at the different irradiation timings, and wherein each of the plurality of light detecting means is adapted to detect light at the detection timing synchronized with the irradiation timing of the corresponding light irradiating means.

2. The scattering medium measuring apparatus according to claim 1, wherein the interval of the irradiation timing between two of the light irradiating means having successive irradiation timings is $1\mu$sec or less.

3. The scattering medium measuring apparatus according to claim 1 or 2 , wherein N pieces of light sources (11, 21)are installed to supply pulse light, respectively, to N pieces of the light irradiating means.

4. The scattering medium measuring apparatus according to claims 1 or 2, wherein M pieces (M represents an integer of 1 or more to less than N) of light sources (11, 21) are installed to supply pulse light to a plurality of light irradiating means among N pieces of the light irradiating means.

5. The scattering medium measuring apparatus according to any of claims 1 to 4, wherein part of a plurality of the light detecting means is shared by a plurality of the measuring modules (1, 2).

6. The scattering medium measuring apparatus according to any of claims 1 to 5, wherein the N pieces of measuring modules are arranged adjacent to each other, or arranged in such a manner as to be overlapped partially with each other.

**7.** The scattering medium measuring apparatus according to any of claims 1 to 6, wherein, in each of the N pieces of measuring modules, the detection probes are arranged at each vertex of a regular polygonal shape centering on the irradiation probe (60, 70; 22)

**Patentansprüche**

**1.** Messvorrichtung für streuende Medien zum Durchführen einer Messung unter Verwendung eines zeitaufgelösten Spektroskopieverfahrens, umfassend:

N Stück (N bezeichnet eine Ganzzahl von 2 oder mehr) Messmodule (1, 2), welche jeweils aus einem Licht-Ausstrahlmittel zum Ausstrahlen von gepulstem Licht, welches von einer vorbestimmten Licht-Ausstrahlposition ($P_{10}$, $P_{20}$) ausgestrahlt wird, in ein streuendes Mediums (SM) zum nicht-invasiven Messen von internen Informationen davon, sowie einer Mehrzahl von Licht-Detektionsmitteln zum Detektieren von von den Licht-Ausstrahlmitteln ausgestrahltem und durch das Innere des streuenden Mediums (SM) propagiertem Licht an vorbestimmten Licht-Detektionspositionen ($P_{11}$, $P_{12}$; $P_{21}$, $P_{22}$) bestehen, **gekennzeichnet durch**:

Zeitgabe-Anweisungsmittel (50) zum Anweisen der Licht-Ausstrahlmittel und der Mehrzahl von Licht-Detektionsmitteln, welche jeweils in jedem der N Stück Messmodule (1, 2) umfasst sind, über eine Ausstrahlungs-Zeitgabe und eine Detektions-Zeitgabe, wobei in jedem der N Stück Messmodule (1, 2) eine Ausstrahlungs-Sonde (60, 70; 22) der Licht-Ausstrahlmittel im Zentrum des Messmoduls (1, 2) positioniert ist, Detektions-Sonden (61a, 71a, 81a, 91a; 41a) der Mehrzahl von Licht-Detektionsmitteln um die Ausstrahlungs-Sonde (60, 70; 22) herum angeordnet sind und die Ausstrahlungs-Sonde (60, 70; 22) und die Detektions-Sonden (61a, 71a, 81a, 91a; 41a) derart angeordnet sind, dass jede Licht-Detektionsposition ($P_{11}$, $P_{12}$; $P_{21}$, $P_{22}$) von der Licht-Ausstrahlposition ($P_{10}$, $P_{20}$) den gleichen Abstand aufweist, und wobei auf Grundlage von Anweisungs-Signalen von den Zeitgabe-Anweisungsmitteln (50) N Stück der Licht-Ausstrahlmittel, welche jeweils den N Stück Messmodulen (1, 2) entsprechen, dazu eingerichtet sind, in das streuende Medium (SM) das gepulste Licht nacheinander zu den verschiedenen Einstahlungs-Zeitgaben einzustrahlen, und wobei jedes aus der Mehrzahl von Licht-Detektionsmitteln dazu eingerichtet ist, Licht zu der Detektions-Zeitgabe zu detektieren, welche mit der Ausstrahlungs-Zeitgabe des entsprechenden Licht-Ausstrahlmittels synchronisiert ist.

**2.** Messvorrichtung für streuende Medien nach Anspruch 1, wobei das Intervall der Ausstrahlungs-Zeitgabe zwischen zwei der Licht-Ausstrahlmittel mit aufeinander folgenden Zeitgaben 1 $\mu$s oder weniger beträgt.

**3.** Messvorrichtung für streuende Medien nach Anspruch 1 oder 2, wobei N Stück Lichtquellen (11, 21) zum Liefern von gepulstem Licht jeweils an N Stück der Licht-Ausstrahlmittel installiert sind.

**4.** Messvorrichtung für streuende Medien nach Anspruch 1 oder 2, wobei M Stück (M bezeichnet eine Ganzzahl von 1 oder mehr bis kleiner als N) von Lichtquellen (11, 21) zum Liefern von gepulstem Licht an einer Mehrzahl von Licht-Ausstrahlmitteln aus N Stück der Licht-Ausstrahlmittel installiert sind.

**5.** Messvorrichtung für streuende Medien nach einem der Ansprüche 1 bis 4, wobei ein Teil aus einer Mehrzahl der Licht-Detektionsmittel von einer Mehrzahl der Messmodule (1, 2) geteilt wird.

**6.** Messvorrichtung für streuende Medien nach einem der Ansprüche 1 bis 5, wobei die N Stück Messmodule benachbart zueinander angeordnet sind oder derart angeordnet sind, dass sie sich teilweise miteinander überlappen.

**7.** Messvorrichtung für streuende Medien nach einem der Ansprüche 1 bis 6, wobei in jedem der N Stück Messmodule die Detektions-Sonden an jeder Ecke einer gleichseitigen Polygonform angeordnet sind, welche die Ausstrahlungs-Sonde (60, 70; 22) zentriert.

**Revendications**

**1.** Appareil de mesure de milieu de diffusion pour effectuer une mesure en utilisant un procédé de spectroscopie à résolution temporelle comprenant
N éléments (N représente un nombre entier supérieur ou égal à 2) de modules de mesure (1, 2) chacun étant constitué d'un moyen d'irradiation de lumière pour irradier un milieu de diffusion (SM) avec une lumière pulsée irradiée à partir d'une position d'irradiation de lumière prédéterminée ($P_{10}$, $P_{20}$) pour mesurer des informations internes de celui-ci de manière non invasive, et une pluralité de moyens de détection de lumière pour détecter la lumière irradiée par le moyen d'irradiation de lumière et se propageant à l'intérieur du milieu de diffusion (SM) à des positions de détection de lumière prédéterminées ($P_{11}$, $P_{12}$ ; $P_{21}$, $P_{22}$) **caractérisé par** :

un moyen d'instruction de moment (50) pour donner une instruction au moyen d'irradiation de lumière et à la pluralité de moyens de détection de lumière inclus dans chacun des N éléments de modules de mesure (1, 2), respectivement, concernant un moment d'irradiation et un moment de détection, où

dans chacun des N éléments de modules de mesure (1, 2), une sonde d'irradiation (60, 70 ; 22) du moyen d'irradiation de lumière est positionnée au centre du module de mesure (1, 2), des sondes de détection (61a, 71a, 81a, 91a ; 41a) de la pluralité de moyens de détection de lumière sont agencées autour de la sonde d'irradiation (60, 70 ; 22) et la sonde d'irradiation (60, 70 ; 22) et les sondes de détection (61a, 71a, 81a, 91a ; 41a) sont agencées de sorte que chaque position de détection de lumière ($P_{11}$, $P_{12}$ ; $P_{21}$, $P_{22}$) ait la même distance de la position d'irradiation de lumière ($P_{10}$, $P_{20}$) et où

sur la base de signaux d'instruction provenant du moyen d'instruction de moment (50), N éléments de moyens d'irradiation de lumière correspondant, respectivement, aux N éléments de modules de mesure (1, 2) sont adaptés pour irradier le milieu de diffusion (SM) avec la lumière pulsée successivement à différents moments d'irradiation, et où chacun de la pluralité de moyens de détection de lumière est adapté pour détecter la lumière au moment de détection synchronisé avec le moment d'irradiation du moyen d'irradiation de lumière correspondant.

2. Appareil de mesure de milieu de diffusion selon la revendication 1, dans lequel l'intervalle du moment d'irradiation entre deux moyens parmi les moyens d'irradiation de lumière ayant des moments d'irradiation successifs est inférieur ou égal à 1 $\mu$sec.

3. Appareil de mesure de milieu de diffusion selon la revendication 1 ou 2, dans lequel N éléments de sources de lumière (11, 21) sont installés pour fournir une lumière pulsée, respectivement, aux N éléments de moyens d'irradiation de lumière.

4. Appareil de mesure de milieu de diffusion selon la revendication 1 ou 2, dans lequel M éléments (M représente un nombre entier supérieur ou égal à 1 mais inférieur à N) de sources de lumière (11, 21) sont installés pour fournir une lumière pulsée à une pluralité de moyens d'irradiation de lumière parmi les N éléments de moyens d'irradiation de lumière.

5. Appareil de mesure de milieu de diffusion selon l'une des revendications 1 à 4, dans lequel une partie d'une pluralité de moyens de détection de lumière est partagée par une pluralité de modules de mesure (1, 2).

6. Appareil de mesure de milieu de diffusion selon l'une des revendications 1 à 5, dans lequel les N éléments de modules de mesure sont agencés de manière adjacente les uns aux autres, ou agencés de manière à se chevaucher partiellement les uns avec les autres.

7. Appareil de mesure de moyen de diffusion selon l'une des revendications 1 à 6, dans lequel, dans chacun des N éléments de modules de mesure, les sondes de détection sont agencées au niveau de chaque sommet d'une forme polygonale régulière centrée sur la sonde d'irradiation (60, 70 ; 22).

*Fig.1*

Fig.2

EP 1 653 218 B1

# Fig.3

(a) PULSE LIGHT (MODULES 1,2)

$S_{t0}$, $A_1$, $A_2$, $2\Delta T$, TIME t

(b) SIGNAL (MODULE 1)

$T_0$, $T_1$, $C_1$, $B_1$, TIME t

(c) SIGNAL (MODULE 2)

$\Delta T$, $T_0$, $T_2$, $C_2$, $B_2$, TIME t

(d) WAVEFORM (MODULE 1)

$D_1$, TIME t

# *Fig.4*

# Fig.5

## Fig.6

1B    3B

101    301    103    303

100    203

201    401    300    403

200    104

102    302    400    304

202    402    204    404

2B    4B

# Fig.7

(a)

(b)

2C

1C

1D

2D

(c)

3E

1E

2E

# Fig.8

# Fig.9

# Fig.10

(a)

(b)

# Fig.11

(a)

(b)

# Fig.12

## Fig.13

EP 1 653 218 B1

# Fig.14

(a)

$P_1$ $P_0$

SM

$t=t_1$

(b)

$P_1$ $P_0$

SM

$t=t_2$

(c)

$P_1$ $P_0$

SM

$t=t_3$

# Fig.15

(a)

(b)

(c)

(d)

*Fig.16*

# Fig.17

(a) PULSE LIGHT (MODULE 1)

$S_{t1}$

$2\Delta T$

$A_1$

TIME t

(b) SIGNAL (MODULE 1)

$T_0$

$T_1$

$C_1$  $B_1$

TIME t

$\Delta T$

(c) PULSE LIGHT (MODULE 2)

$S_{t2}$

$A_2$

TIME t

(d) SIGNAL (MODULE 2)

$T_0$

$T_2$

$C_2$  $B_2$

TIME t

# Fig.18

## Fig.19

EP 1 653 218 B1

# Fig.20

EP 1 653 218 B1

# *Fig.21*

(a)

(b)

(c)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI09184800 B
- JP 2001178708 A
- JP 9184800 A
- EP 0758082 A
- US 5090415 A
- US 6397099 B
- US 20030030809 A

**Non-patent literature cited in the description**

- **H.EDA et al.** Multichannel time-resolved optical tomographic imaging system. *Review of Scientific Instruments,* 1999, vol. 70, 3595
- **H.EDA et al.** *Review of Scientific Instruments,* 1999, vol. 70, 3595